# EUROPÄISCHE PATENTANMELDUNG

(11) **EP 1 203 821 A2**
(43) Veröffentlichungstag der Anmeldung: **08.05.2002**
(21) Anmeldenummer: 01125184.0
(22) Anmeldetag: 24.10.2001
(51) Int. Cl.: C12P 13/14, C12P 13/04, C12P 17/00, C12P 17/02, C12P 17/10, C12P 17/14, C12P 7/50, C12P 7/62, A61K 31/198, A61K 7/00

(54) **Reduktive Aminierung von alpha-Ketodicarbonsäurederivaten**

(30) Priorität: 03.11.2000 DE 10054492
(71) Anmelder: Degussa Aktiengesellschaft, 40474 Düsseldorf (DE)
(72) Erfinder: Rossen, Kai, Dr., 63452 Hanau (DE); Sarich, Martin, 63755 Alzenau (DE); Latinovic, Milan, 63667 Nidda (DE); Rollmann, Claudia, 63755 Alzenau (DE); Bommarius, Andreas, Prof., 30327 Atlanta, GA (US)

(57) **Zusammenfassung**

Die Erfindung betrifft ein enzymatisches Verfahren zur reduktiven Aminierung von α-Ketodicarbonsäurederivaten der allgemeinen Formel I oder deren Salze.

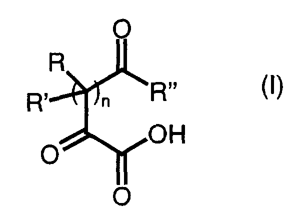

Die reduktive Aminierung wird mittels einer Aminosäuredehydrogenase insbesondere einer Phenylalanindehydrogenase durchgeführt.

Verwendung der so hergestellten Verbindungen.

## Beschreibung

Die vorliegende Erfindung richtet sich auf ein enzymatisches Verfahren zur reduktiven Aminierung von Ketodicarbonsäurederivaten der allgemeinen Formel I oder deren Salze.

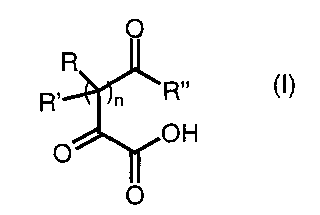

Die Produkte dieser Reaktion sind spezielle hoch enantiomerenangereicherte α-Aminodicarbonsäurederivate, welche als Vorstufen in der Synthese bioaktiver Wirkstoffe, insbesondere Arzneimitteln zum Einsatz kommen (N. Moss et al., Synthesis 1997, 32ff).

Die ins Auge gefaßten Moleküle können durch klassische chemische Synthese hergestellt werden, doch erfordert dieses die Anwendung von toxischen Metallen (Blei) oder metallorganischen Reagenzien bei tiefen Temperaturen (J. Org. Chem. 1999, 64, 4362ff; J. Org. Chem. 1990, 55, 3068ff). Beide Reagenzien sind für den Einsatz in technischem Maßstab kontraproduktiv.

Im Stand der Technik ist auch bekannt, daß die enzymatische reduktive Aminierung von α-Ketocarbonsäuren mit Aminsäuredehydrogenasen als Biokatalysatoren erfolgen kann. Derartige Enzyme sind im technischen Einsatz erprobt (J. Biotechn. 1997, 53, 29; J. Org. Chem. 1990, 55, 5567; Enzyme Catalysis in Organic Synthesis, Eds.: K. Drauz and H. Waldmann, VCH, 1995, 633ff). Die ins Auge gefaßten Verbindungen wurden aber bisher noch nicht mit Aminosäuredehydrogenase umgesetzt.

Von einer Leucindehydrogenase (LeuDH) ist bekannt, daß sie eher aliphatisch substituierte minder sperrige α-Ketosäuren als Substrate akzeptiert, während eine Phenylalanindehydrogenase (PheDH) besser solche Substrate umsetzt, die in α-Stellung sehr raumerfüllende hydrophobe Substituenten aufweisen.
Allerdings geht aus J. Biotechn. 1997, 53, 29 hervor, daß die 2-Oxo-3,3-dimethylbuttersäure ein schlechtes Substrat für die dort verwendete PheDH ist.

Aufgabe der vorliegenden Anmeldung war es, ein enzymatisch arbeitendes Verfahren zur Verfügung zu stellen, mit dem man α-Ketodicarbonsäurederivate reduktiv aminieren kann, welche in Nachbarstellung zur Ketogruppe ein tertiäres C-Atom aufweisen. Weiterhin sollte das Verfahren allen Anforderungen an eine Durchführung im technischen Maßstab genügen, insbesondere sollte es sowohl in ökologischer wie ökonomischer Hinsicht vorteilhaft sein.

Gelöst wird diese Aufgabe durch ein Verfahren mit den Merkmalen des Anspruchs 1. Vorteilhafte Ausgestaltungen des erfindungsgemäßen Verfahrens finden sich in den von Anspruch 1 abhängigen Unteransprüchen. Anspruch 9 schützt eine bevorzugte Verwendung der erfindungsgemäß hergestellten Verbindungen.

Dadurch, daß man für ein Verfahren zur reduktiven Aminierung von α-Ketodicarbonsäurederivaten der allgemeinen Formel I oder deren Salze 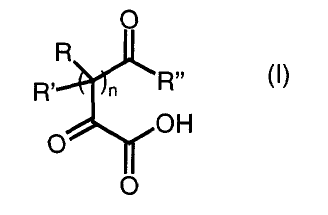 worin
n = 1-3 ist,
R, R' unabhängig voneinander bedeuten
H, (C₁-C₈)-Alkyl, (C₁-C₈)-Alkoxy, (C₂-C₈)-Alkoxyalkyl, (C₆-C₁₈)-Aryl, (C₇-C₁₉)-Aralkyl, (C₃-C₁₈)-Heteroaryl, (C₄-C₁₉)-Heteroaralkyl, (C₁-C₈)-Alkyl-(C₆-C₁₈)-Aryl, (C₁-C₈)-Alkyl-(C₃-C₁₈)-Heteroaryl, (C₃-C₈)-Cycloalkyl, (C₁-C₈)-Alkyl-(C₃-C₈)-Cycloalkyl, (C₃-C₈)-Cycloalkyl- (C₁-C₈)-Alkyl,
oder R und R' formen zusammen ein Ring über eine (C₂-C₅)-Alkylenbrücke, welche eine oder mehrere Doppelbindungen enthalten und/oder einfach oder mehrfach mit (C₁-C₈)-Alkyl, (C₁-C₈)-Acyl, (C₁-C₈)-Alkoxy, (C₂-C₈)-Alkoxyalkyl substituiert und/oder im Ring Heteroatome wie N, O, P, S enthalten kann,
mit der Maßgabe, daß die an verschiedene C-Atome gebundenen R und R' unabhängig voneinander sind und in Nachbarstellung zur Ketofunktion können R, R' nicht H sein,
R'' bedeutet
OR, NHR, NRR', wobei in diesen Fällen R bzw. R' nicht (C₁-C₈)-Alkoxy sein kann,
eine Aminosäuredehydrogenase verwendet, gelangt man völlig überraschend in dafür aber nicht minder vorteilhafter Weise zu den gewünschten hoch enantiomerenangereicherten Aminosäureprodukten.

Vorzugsweise erfolgt die erfindungsgemäße Umsetzung mit einer Leucin- (LeuDH) oder Phenylalanindehydrogenase (PheDH). Hierbei ist insbesondere der Aspekt als überraschend zu werten, daß eine LeuDH oder PheDH, welche im allgemeinen nur hydrophobe Seitenketten in Substraten akzeptieren auch hydrophile Reste wie Säuren und deren Salze, Ester oder Amide zuläßt. Dies war ausgehend vom Stand der Technik nicht zu erwarten.

Im Prinzip ist es dem Fachmann überlassen, welche Aminosäuredehydrogenase er für die erfindungsgemäße Reaktion einsetzt. Vorzugsweise wird eine solche verwendet, die im Hinblick auf die Anwendung der Reaktion im technischen Maßstab am besten geeignet ist. Sie sollte deshalb u.a. hinsichtlich Umsatz, Stabilität und Verfügbarkeit möglichst optimal sein. Vorzugsweise wird eine PheDH oder LeuDH verwendet. Besondersbevorzugt ist die PheDH der Organismen, welche in US5,851,810, J. Biotechn. 1997, 53, 29 und J. Org. Chem. 1990, 55, 5567 genannt sind. Besonders bevorzugt verwendet man jedoch die PheDH aus Rhodococcus M4 (DSM3041; US5,416,019; Seq. 2).

Weiter vorzugsweise werden in dem erfindungsgemäßen Verfahren Verbindungen der allgemeinen Formel I oder deren Salze eingesetzt, in denen
n = 1 ist oder
R, R' (C₁-C₈)-Alkyl ist oder
R und R' formen zusammen ein Ring über eine (C₂-C₅)-Alkylenbrücke oder
R'' OH, NH₂ ist.

Aminosäuredehydrogenasen, insbesondere Leucindehydrogenasen und Phenylalanindehydrogenasen sind Coenzym-abhängige (NADH/NADPH) Enzyme (Beyer, Walter, Lehrbuch der organischen Chemie, 22. Auflage, S.Hirzel Verlag Stuttgart, S. 886). Im technischen Maßstab hat es sich als vorteilhaft erwiesen, das Coenzym zu regenerieren (Enzyme Catalysis in Organic Synthesis, Eds.: K. Drauz and H. Waldmann, VCH, 1995, 596ff), insbesondere mittels einer FDH und einer Formiatquelle, um dadurch die Einsatzmenge an NADH/NADPH reduzieren zu können (J. Biotechn. 1997, 53, 29).
Als Formiatquelle können die dem Fachmann für diesen Zweck bekannten Substanzen verwendet werden (Enzyme Catalysis in Organic Synthesis, Eds.: K. Drauz and H. Waldmann, VCH, 1995, S.596). Dazu zählt vorzugsweise ein Salz der Ameisensäure, besonders bevorzugt ist Ammoniumformiat.
Die FDH kann vom Fachmann frei gewählt werden. In der Literatur sind verschiedenste FDHs bekannt (DE19753350.7).

Vorzugsweise wird für die betrachtete Reaktion die FDH aus C. boidinii verwendet, ggf. in ihrer durch Mutationen bedingten stabileren Form.
Die Mengen an Einsatzstoffen und Enzymen bei der vorliegenden Reaktion bewegen sich in einem unter wirtschaftlichen Gesichtspunkten vernünftigen Rahmen, vorteilhafterweise jedoch zwischen den in der unten stehenden Tabelle aufgeführten Werten.

**Tabelle:**

| Mengen von Einsatzstoffen und Enzymen (diese sind bezogen auf 1 g der Verbindung der allgemeinen Formel I) | | | |
|---|---|---|---|
| Einsatzstoff/Enzym | Min.-Wert | Max.-Wert | bevorzugt. Bereich |
| Formiat | 0,1 mol/l | 3,0 mol/l | 1-2,0 mol/l |
| FDH | 2 U | 50 U | 10-30 U |
| NADH/NADPH | 1 mg | 1 g | 2-20 mg |
| PheDH | 1 U | 100 U | 5-25 U |

Die Verbindung der allgemeinen Formel I selbst wird vorteilhafterweise in einer Konzentration von 0,05 mol/l-3,0 mol/l, vorzugsweise 0,5 mol/l-1,5 mol/l, in der reduktiven Aminierung eingesetzt.
Diese wird ökologisch vorteilhaft in Wasser als Lösungsmittel durchgeführt. Der Zusatz von wasserlöslichen organischen Solventien kann aus Löslichkeitsgründen indiziert sein. In diesem Fall wird vorzugsweise Methanol, Ethanol, Aceton, Eisessig (vorzugsweise bis 10%) zur Reaktionsmischung dazugegeben.
Der pH-Wert bei der Reaktion sollte zwischen 7,5 und 10,0 vorzugsweise zwischen 8,0 und 9,0 liegen. Ganz besonders bevorzugt arbeitet man bei einem pH-Wert von 8,4.

Die Temperatur bei der Reaktion sollte nicht zu hoch gewählt werden, um die Funktionsfähigkeit der Enzyme nicht zu gefährden. Auf der anderen Seite darf die Temperatur nicht zu niedrig sein, da die Reaktion sonst zu langsam vonstatten geht. Vorteilhafterweise liegt die Temperatur der Reaktion bei +15 bis +50°C, vorzugsweise zwischen +30 und +40°C.

Besonders bevorzugt erfolgt der erfindungsgemäße Prozeß in einem Enzym-Membran-Reaktor (DE 199 10 691.6).

Die genannten Enzyme können in freier Form als homogen aufgereinigte Verbindungen oder als rekombinant hergestellte Enzyme verwendet werden. Weiterhin können die Enzyme auch als Bestandteil eines intakten (Gast-)Organismus eingesetzt werden oder in Verbindung mit der aufgeschlossenen und beliebig weit aufgereinigten Zellmasse des jeweiligen Wirtsorganismus. Möglich ist ebenfalls die Verwendung der Enzyme in immobilisierter Form (Bhavender P. Sharma, Lorraine F. Bailey and Ralph A. Messing, "Immobilisierte Biomaterialiern - Techniken und Anwendungen", Angew. Chem. 1982, 94, 836-852). Vorteilhafterweise erfolgt die Immobilisierung durch Lyophilisation (Dordick et al. J. Am. Chem. Soc. 194, 116, 5009-5010; Okahata et al. Tetrahedron Lett. 1997, 38, 1971-1974; Adlercreutz et al. Biocatalysis 1992, 6, 291-305). Ganz besonders bevorzugt ist die Lyophilisation in Gegenwart von oberflächenaktiven Substanzen, wie Aerosol OT oder Polyvinylpyrrolidon oder Polyethylenglycol (PEG) oder Brij 52 (Diethylenglycol-mono-cetylether) (Goto et al. Biotechnol. Techniques 1997, 11, 375-378). Die Verwendung als CLECs ist ebenfalls denkbar (Vaghjiani et al., Biocat. Biotransform. 2000, 18, 157ff).

Ein weiterer Aspekt der Erfindung betrifft die Verwendung der nach einem oder mehreren der vorhergehenden Ansprüchen hergestellten Verbindungen in einem Verfahren zur Herstellung von bioaktiven Wirkstoffen, vorzugsweise Arzneimitteln.

Die Herstellung der Ausgangsverbindungen erfolgt nach dem Fachmann bekannten Verfahren (J. Biotechn. 1997, 53, 29; J. Org. Chem. 1990, 55, 5567). Generell lassen sich die α-Ketodicarbonsäurederivate jedoch besonders vorteilhaft wie folgt darstellen:

Acetylcarbonsäurederivate werden mit zur elektrophilen Substitution befähigten z.B. haloorganischen Verbindungen (RHal, R'Hal) in Gegenwart einer Base in einem indifferenten organischen Lösungsmittel umgesetzt. Anschließend erfolgt die Oxidation der endständigen Methylfunktion zur Säure.
Die erhaltenen α-Ketodicarbonsäurederivate werden sodann in Wasser mit den Enzymen in Gegenwart einer Formiatquelle kontaktiert. Die betrachtete Reaktion verläuft in der Regel quantitativ und mit hoher chiraler Induktion (Schema).

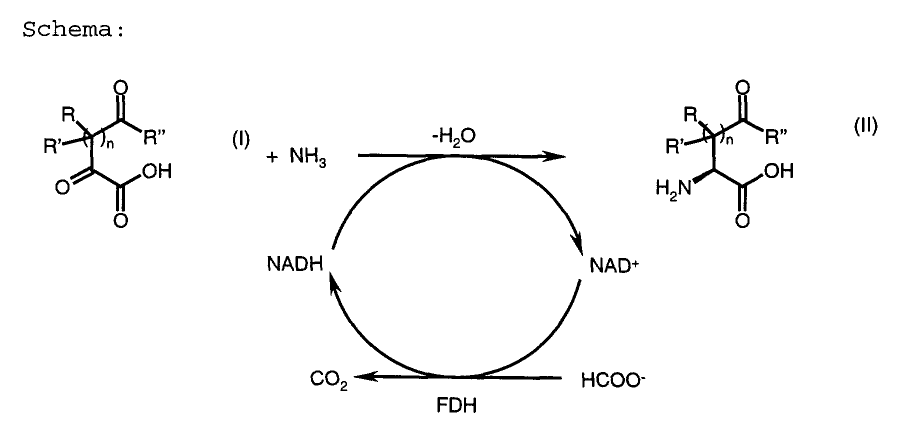

Die Aminosäure kann nach dem Fachmann bekannten Verfahren aufgearbeitet werden. Vorzugsweise erfolgt die Isolierung der Aminosäure durch Ultrafiltration mit anschließender Ionenaustauschchromatographie oder Kristallisation. Derartige Verfahren sind dem Fachmann bekannt (Houben-Weyl, Band E16d, Georg Thieme Verlag Stuttgart, 1992, S.406ff). Als (C₁-C₈)-Alkylreste sind anzusehen Methyl, Ethyl, *n*-Propyl, Isopropyl, *n*-Butyl, Isobutyl, *sec*-Butyl, *tert*-Butyl, Pentyl, Hexyl, Heptyl oder Octyl samt aller ihrer Bindungsisomeren. Der Rest (C₁-C₈)-Alkoxy entspricht dem Rest (C₁-C₈)-Alkyl mit der Maßgabe, daß dieser über ein Sauerstoffatom an das Molekül gebunden ist. Als (C₂-C₈)-Alkoxyalkyl sind Reste gemeint, bei denen die Alkylkette durch mindestens eine Sauerstoffunktion unterbrochen ist, wobei nicht zwei Sauerstoffatome miteinander verbunden sein können. Die Anzahl der Kohlenstoffatome gibt die Gesamtzahl der im Rest enthaltenen Kohlenstoffatome an. Eine (C₂-C₅)-Alkylenbrücke ist eine Kohlenstoffkette mit zwei bis fünf C-Atomen, wobei diese Kette über zwei verschiedene ihrer C-Atome an das betrachtete Molekül gebunden ist.
Die eben beschriebenen Reste können einfach oder mehrfach mit Halogenen und/oder N-, O-, P-, S-, Si-atomhaltigen Resten substituiert sein. Dies sind insbesondere Alkylreste der oben genannten Art, welche eines oder mehrere dieser Heteroatome in ihrer Kette aufweisen bzw. welche über eines dieser Heteroatome an das Molekül gebunden sind.

Unter (C₃-C₈)-Cycloalkyl versteht man Cyclopropyl, Cyclobutyl, Cyclopentyl, Cyclohexyl bzw. Cycloheptylreste etc. Diese können mit einem oder mehreren Halogenen und/oder N-, O-, P-, S-, Si-atomhaltige Reste substituiert sein und/oder N-, O-, P-, S-Atome im Ring aufweisen, wie z. B. 1-, 2-, 3-, 4-Piperidyl, 1-, 2-, 3-Pyrrolidinyl, 2-, 3-Tetrahydrofuryl, 2-, 3-, 4-Morpholinyl.

Ein (C₃-C₈)-Cycloalkyl-(C₁-C₈)-Alkylrest bezeichnet einen wie oben dargestellten Cycloalkylrest, welcher über einen wie oben angegebenen Alkylrest an das Molekül gebunden ist.

(C₁-C₈)-Acyloxy bedeutet im Rahmen der Erfindung einen wie oben definierten Alkylrest mit max. 8 C-Atomen, welcher über eine COO-Funktion an das Molekül gebunden ist.

(C₁-C₈)-Acyl bedeutet im Rahmen der Erfindung einen wie oben definierten Alkylrest mit max. 8 C-Atomen, welcher über eine CO-Funktion an das Molekül gebunden ist.

Unter einem (C₆-C₁₈)-Arylrest wird ein aromatischer Rest mit 6 bis 18 C-Atomen verstanden. Insbesondere zählen hierzu Verbindungen wie Phenyl-, Naphthyl-, Anthryl-, Phenanthryl-, Biphenylreste oder an das betreffende Molekül annelierte Systeme der vorbeschriebenen Art, wie z.B. Indenylsysteme, welche ggf. mit (C₁-C₈)-Alkyl, (C₁-C₈)-Alkoxy, N(C₁-C₈)-Alkyl, (C₁-C₈)-Acyl, (C₁-C₈)-Acyloxy substituiert sein können.

Ein (C₇-C₁₉)-Aralkylrest ist ein über einen (C₁-C₈)-Alkylrest an das Molekül gebundener (C₆₋C₁₈)-Arylrest.

Ein (C₃-C₁₈)-Heteroarylrest bezeichnet im Rahmen der Erfindung ein fünf-, sechs- oder siebengliedriges aromatisches Ringsystem aus 3 bis 18 C-Atomen, welches Heteroatome wie z. B. Stickstoff, Sauerstoff oder Schwefel im Ring aufweist. Als solche Heteroaromaten werden insbesondere Reste angesehen, wie 1-, 2-, 3-Furyl, wie 1-, 2-, 3-Pyrrolyl, 1-, 2-, 3-Thienyl, 2-, 3-, 4-Pyridyl, 2-, 3-, 4-, 5-, 6-, 7-Indolyl, 3-, 4-, 5-Pyrazolyl, 2-, 4-, 5-Imidazolyl, Acridinyl, Chinolinyl, Phenanthridinyl, 2-, 4-, 5-, 6-Pyrimidinyl.

Unter einem (C₄-C₁₉)-Heteroaralkyl wird ein dem (C₇-C₁₉)-Aralkylrest entsprechendes heteroaromatisches System verstanden.

Als Halogene (Hal) kommen Fluor, Chlor, Brom und Iod in Frage.

### Beispiele:

### Darstellung von 1-Acetylcyclopropancarbonsäureethylester

| | | |
|---|---|---|
| 215 mmol | Ethylacetoacetat | 28.55 g |
| 260 mmol | Dibromethan | 49.34 g |
| 1.075 mol | Kaliumcarbonat | 150 g |
| 650 mL | Dimethylsulfoxid | 650 mL |

Zu einer Lösung von Ethylacetoacetat und Dibromethan in DMSO wird Kaliumcarbonat gegeben. Die Suspension wird 18 Std. bei 25°C gerührt. Nach Zugabe von 550 mL H₂O wird das Produkt in 300 mL MTBE extrahiert. Nach zwei wäßrigen Extraktionen der organischen Phase wird diese produkthaltige Phase eingedampft.

Das Rohprodukt wird dann in 100 mL H₂O und 100 mL MeOH aufgelöst und durch Zugabe von 8 g NaOH über Nacht zur Carbonsäure verseift. Die nach Verdampfen des MeOH erhaltene Lösung wird direkt in der anschließenden Oxidation verwendet.

| | | |
|---|---|---|
| 400 mmol | KMnO₄ | 63 g |
| 400 mmol | NaOH | 16 g |
| 300 mL | | H₂O |

Zu einer Lösung von KMnO₄ und NaOH in H₂O wird die wäßrige Lösung des Methylketons unter Eiskühlung gegeben und die Lösung wird dann über Nacht bei 25°C nachgerührt. Es wird vom ausgefallenen Braunstein abfiltriert und die so erhaltenen Lösung direkt zur enzymatischen reduktiven Aminierung verwendet (Ausbeute 75% per HPLC).

### Reduktive Aminierung von Keto-cyclo-propyl-aspartat mit PheDH/FDH

| | |
|---|---|
| Keto-cyclopropyl-aspartat (KS) | 0,5M |
| Ammoniumformiat | 1,5M |
| NAD⁺-Trihydrat | 5,6mg/g KS |
| PheDH | 6,75U/g KS |
| FDH | 11,1U/g KS |
| pH | 8,2-8,5 |
| Temp. | 30°C |
| Reaktionszeit | 20 - 30h |

Das Keto-cyclo-propyl-aspartat wird mit Ammoniumformiat unter Rühren in VE-Wasser gelöst. Temperatur und pH-Wert werden eingestellt. PheDH, FDH und NAD⁺-Trihydrat werden zugegeben. Während der gesamten Reaktion wird die Temperatur und der pH-Wert konstant gehalten. Gegebenenfalls muß der pH-Wert durch Zugabe von Ammoniaklösung oder Ameisensäure bzw. den entsprechenden Verdünnungen korrigiert werden.

Nach beendeter Reaktion werden die Enzyme per Ultrafiltration abgetrennt. Die Aminosäure wird nach bekannter Manier durch Ionenaustauschchromatographie isoliert (Ausbeute 99%).

Die Spektrenaufnahme erfolgte an einem DRX 500 MHz NMR-Spektrometer der Firma Bruker (Rheinstetten, Deutschland) bei einer Frequenz für Protonen von 500,13 MHz. Die chemischen Verschiebungen wurden gegen Tetramethylsilan (TMS) als internen Standard referenziert. Die Messungen erfolgten in DMSO-d₆ bei 303 K.

¹H-NMR δ 1.20 (m, 1 H), 1.25 (m, 2 H), 1,34 (m, 1 H), 3.56 (s, 1 H), 8.37 (b, 3 H).

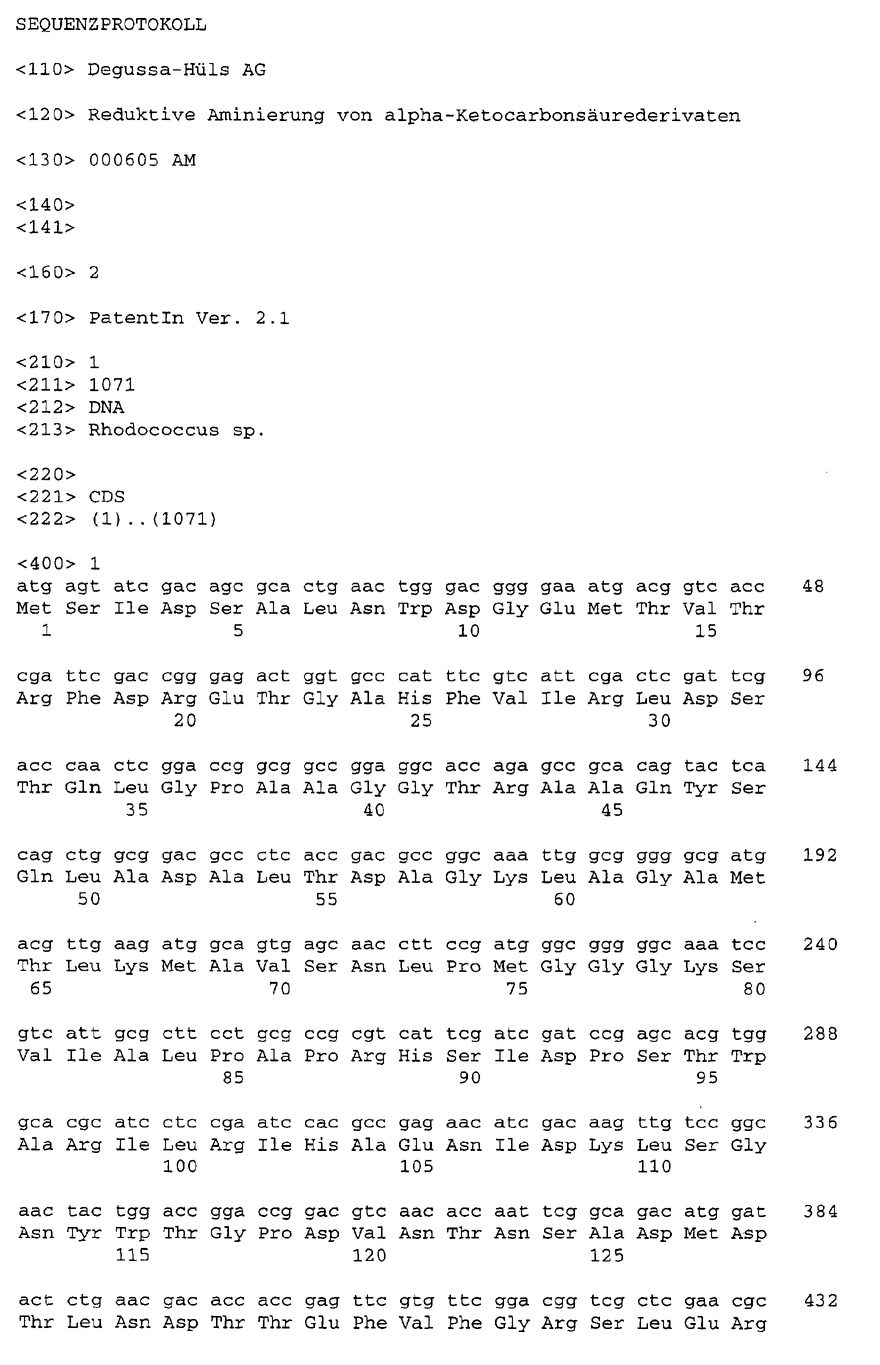

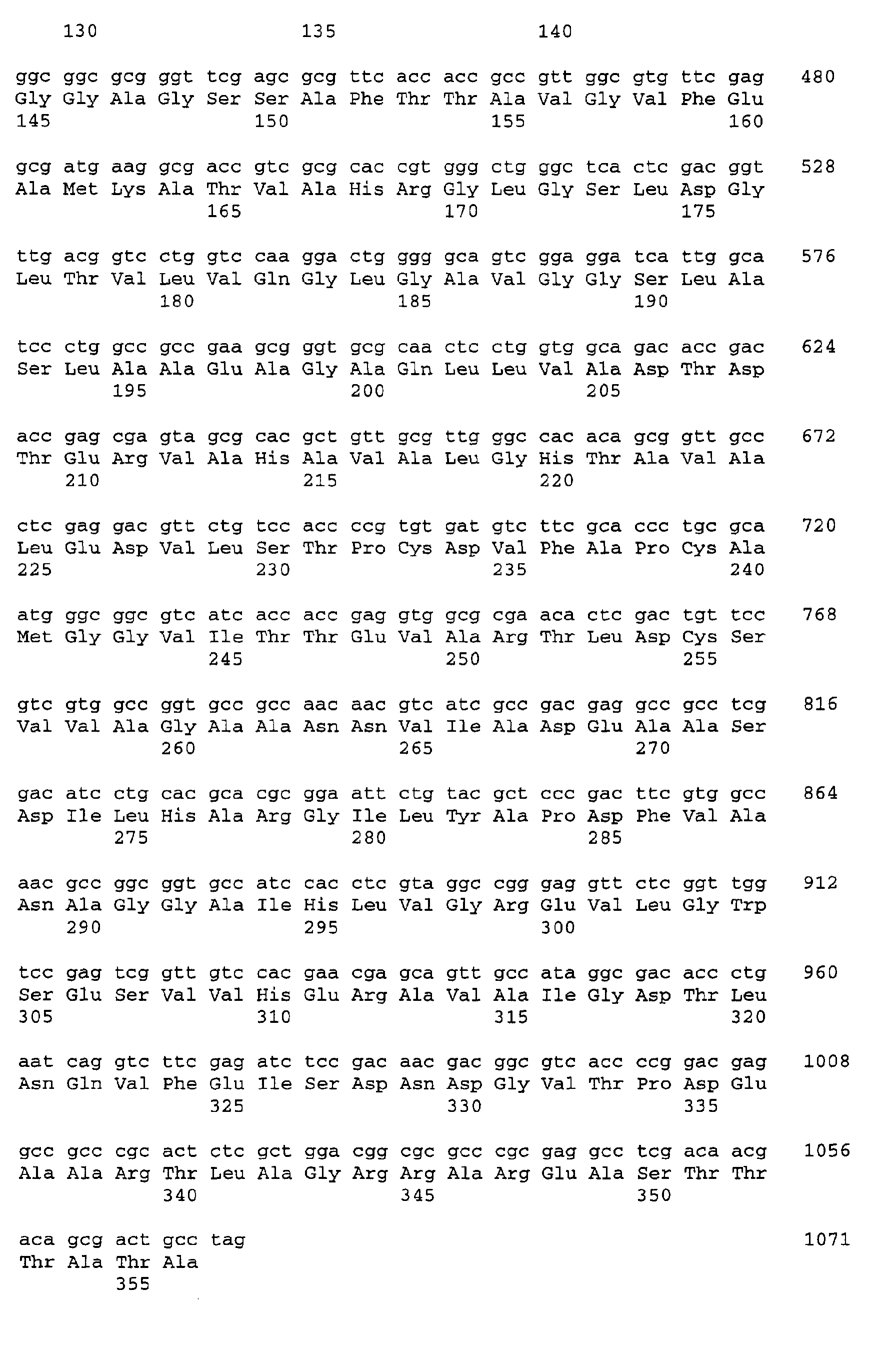

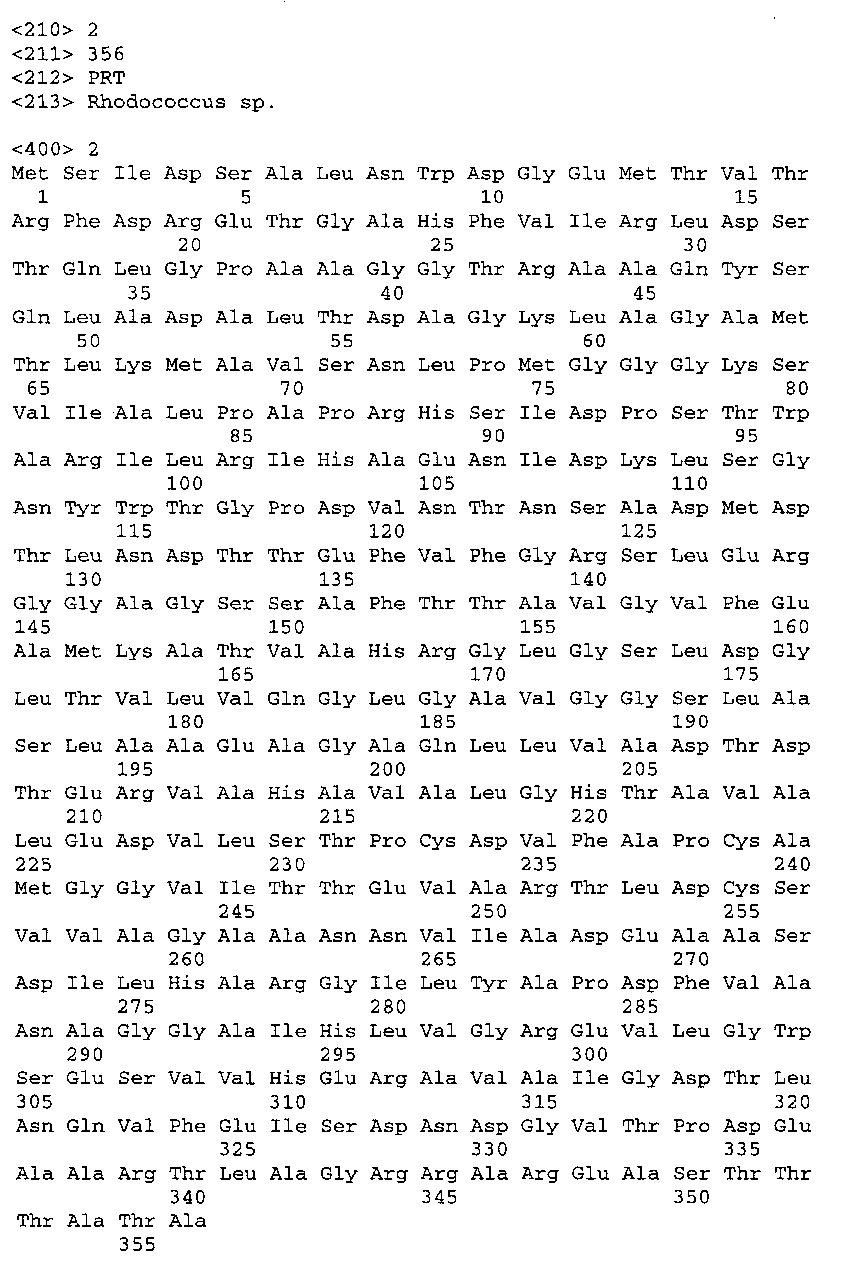

## Patentansprüche

1. Verfahren zur reduktiven Aminierung von α-Ketodicarbonsäurederivaten der allgemeinen Formel I oder deren Salze 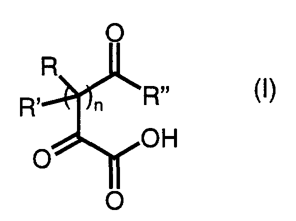 worin
n = 1-3 ist,
R, R' unabhängig voneinander bedeuten
H, (C₁-C₈)-Alkyl, (C₁-C₈)-Alkoxy, (C₂-C₈)-Alkoxyalkyl, (C₆-C₁₈)-Aryl, (C₇-C₁₉)-Aralkyl, (C₃-C₁₈)-Heteroaryl, (C₄-C₁₉)-Heteroaralkyl, (C₁-C₈)-Alkyl- (C₆-C₁₈)-Aryl, (C₁-C₈)-Alkyl- (C₃-C₁₈)-Heteroaryl, (C₃-C₈)-Cycloalkyl, (C₁-C₈)-Alkyl-(C₃-C₈)-Cycloalkyl, (C₃-C₈)-Cycloalkyl- (C₁-C₈)-Alkyl,
oder R und R' formen zusammen ein Ring über eine (C₂-C₅)-Alkylenbrücke, welche eine oder mehrere Doppelbindungen enthalten und/oder einfach oder mehrfach mit (C₁-C₈)-Alkyl, (C₁-C₈)-Acyl, (C₁-C₈)-Alkoxy, (C₂-C₈)-Alkoxyalkyl substituiert und/oder im Ring Heteroatome wie N, O, P, S enthalten kann, mit der Maßgabe, daß die an verschiedene C-Atome gebundenen R und R' unabhängig voneinander sind und in Nachbarstellung zur Ketofunktion können R, R' nicht H sein,
R'' bedeutet
OR, NRR', wobei in diesem Fall R bzw. R' nicht (C₁-C₈)-Alkoxy sein kann,
unter Verwendung einer Aminosäuredehydrogenase.

2. Verfahren nach Anspruch 1,
**dadurch gekennzeichnet, daß** die Aminosäuredehydrogenase eine Leucindehydrogenase oder Phenylalanindehydrogenase ist.

3. Verfahren nach Anspruch 1,
**dadurch gekennzeichnet, daß**
in der allgemeinen Formel I oder deren Salze
n = 1 ist,
R, R' (C₁-C₈)-Alkyl ist oder
R und R' formen zusammen ein Ring über eine (C₂-C₅)-Alkylenbrücke,
R'' OH, NH₂ ist.

4. Verfahren nach einem oder mehreren der vorhergehenden Ansprüche,
**dadurch gekennzeichnet, daß**
die reduktive Aminierung in Gegenwart von NADH oder NADPH und einer FDH durchgeführt wird.

5. Verfahren nach einem oder mehreren der vorhergehenden Ansprüche,
**dadurch gekennzeichnet, daß**
die Verbindung der allgemeinen Formel I in einer Konzentration von 0,05-3,0 mol/l, vorzugsweise 0,5-1,5 mol/l, in der reduktiven Aminierung eingesetzt wird.

6. Verfahren nach einem oder mehreren der vorhergehenden Ansprüche,
**dadurch gekennzeichnet, daß**
der pH-Wert bei der Reaktion zwischen 7,5 und 10, vorzugsweise zwischen 8 und 9, liegt.

7. Verfahren nach einem oder mehreren der vorhergehenden Ansprüche,
**dadurch gekennzeichnet, daß**
die Temperatur der Reaktion bei +15° bis +50°C, vorzugsweise zwischen +30° und +40°C, beträgt.

8. Verfahren nach einem oder mehreren der vorhergehenden Ansprüche,
**dadurch gekennzeichnet, daß**
man in einem Enzym-Membran-Reaktor arbeitet.

9. Verwendung der nach einem oder mehreren der vorhergehenden Ansprüche hergestellten Verbindungen in einem Verfahren zur Herstellung von bioaktiven Wirkstoffen, vorzugsweise Arzneimitteln.
